(19) Europäisches Patentamt
European Patent Office
Office européen des brevets

(11) Publication number: **0 200 251 A2**

(12) **EUROPEAN PATENT APPLICATION**

(21) Application number: **86200582.4**

(22) Date of filing: **04.04.86**

(51) Int. Cl.[4]: **C07C 85/06**, C07D 295/02

(30) Priority: **04.04.85 US 720158**

(43) Date of publication of application: **10.12.86 Bulletin 86/45**

(84) Designated Contracting States: **BE DE FR GB IT NL SE**

(71) Applicant: **UNION CARBIDE CORPORATION**
**39 Old Ridgebury Road**
**Danbury Connecticut 06817(US)**

(72) Inventor: **Doumaux, Arthur Roy, Jr.**
**1401 Wilkie Drive**
**Charleston West Virginia 25314(US)**
Inventor: **Schreck, David James**
**5226 Sun Valley Drive**
**Cross Lanes West Virginia 25313(US)**

(74) Representative: **Smulders, Theodorus A.H.J. et al**
**Vereenigde Octrooibureaux Nieuwe Parklaan 107**
**NL-2587 BP 's-Gravenhage(NL)**

# (54) Use of pressure to control the noncyclics/cyclics ration of polyalkylene amines.

(57) A process for producing polyalkylene polyamines by reacting an alkylenamine compound having at least two amino groups and an alkanolamine having at least one amino group. The molecular ratio of produced noncyclic polyalkylene polyamines to produced cyclic polyalkylene polyamines is controlled as desired by controlling the pressure at which the reaction is conducted. For example, raising the pressure increases the mole ratio of noncyclics to cyclics. The reaction is conducted in the presence of a catalytically effective amount of a phosphorus-containing catalyst at a temperature and pressure sufficient to form the polyalkylene polyamines. The reaction is preferably conducted in the gaseous phase.

EP 0 200 251 A2

# USE OF PRESSURE TO CONTROL THE NONCYCLICS/CYCLICS RATIO OF POLYALKYLENE AMINES

## BACKGROUND OF THE INVENTION

The invention relates to the preparation of polyalkylene polyamines.

U. S. Patent No. 4,463,193 discloses a process for preparing predominantly noncyclic polyalkylene polyamines. Ammonia or a primary or secondary amine is contacted with an alkanolamine compound having an amino group and a primary or secondary hydroxy group and an alkyleneamine compound having two amino groups in the presence of a catalytically effective amount of a Group IIIB metal acid phosphate. A temperature is used which is sufficient to effect a reaction among the ammonia or amine, the alkanolamine compound and the alkyleneamine compound under a pressure sufficient to maintain a substantial amount of the ammonia or amine in the reaction zone.

Table 4, for example, of U. S. Patent No. 4,463,193 does not disclose any effect on the mole ratio of noncyclic polyalkylene polyamines to cyclic polyalkylene polyamines.

## BROAD DESCRIPTION OF THE INVENTION

The invention broadly involves controlling the noncyclics/cyclics ratio of polyalkylene polyamines produced by controlling the pressure in the reaction zone.

The invention, more specifically, involves a process for producing polyalkylene polyamines by an alkyleneamine compound having at least two amino groups and an alkanolamine having at least one amino group. A continuous reaction is involved. The molecular ratio of produced noncyclic polyalkylene polyamines to produced cyclic polyalkylene polyamines is controlled as desired by controlling the pressure at which the reaction is conducted, with the other factors and conditions being held constant. The reaction is conducted in the presence of a catalytically effective amount of a phosphorus-containing catalyst at a temperature and pressure sufficient to form these polyalkylene polyamines. The reaction is preferably conducted in the vapor phase or supercritical phase.

When the pressure is increased during the reaction, an increase of the mole ratio of the produced noncyclic polyalkylene polyamines to the produced cyclic polyalkylene polyamines results.

A preferred embodiment of the invention involves increasing the reaction pressure to between 1,000 and 1,500 psig from a lower pressure level, say, about 200 to 500 psig, which causes an increase of the mole ratio of the produced noncyclic polyalkylene polyamines to the produced cyclic polyalkylene polyamines. Noncyclic polyalkylene polyamine selectivities of 80 to 95 percent can thereby be obtained, depending on the conversion which is a function chiefly of temperature and space velocity. Another preferred embodiment of the invention involves increasing the reaction pressure to greater than 1,500 psig from a lower pressure level, which causes an increase of the mole ratio of the produced noncyclic polyalkylene polyamines to the produced cyclic polyalkylene polyamines.

Also, when the pressure is decreased during the reaction, a decrease of the mole rato of the produced noncyclic polyalkylene polyamines to the produced cyclic polyalkylene polyamines occurs. The pressure during the reaction can be decreased by decreasing the temperature of the reaction, whereby the mole ratio is decreased.

Another preferred embodiment of the invention involves decreasing the reaction pressure to between 200 and 300 psig from a higher pressure level, say about 1,000 to 1,400 psig, which causes a decrease of the mole ratio of the produced noncyclic polyalkylene polyamine to the produced cyclic polyalkylene polyamines. Noncyclic polyalkylene polyamine selectivities of 65 to 75 percent can thereby be obtained, depending on the conversion which is a function chiefly of temperature and space velocity.

The pressure of feed can be increased before the reaction is started so as to cause an increase in the level of the mole ratio of the produced noncyclic polyalkylene polyamines to the produced cyclic polyalkylene polyamines throughout the reaction. The increased level of the mole ratio is higher than it would have been without the pressure increase.

The pressure of the feed can be decreased before the reaction is started so as to cause a decrease in the level of the mole ratio of the produced noncyclic polyalkylene polyamines to the produced cyclic polyalkylene polyamines throughout the reaction. The decreased level of the mole ratio is lower than it would have been without the pressure decrease.

The reaction, for example, is thought to proceed by the reaction of ethylenediamine with monoethanolamine to form diethylenetriamine and a molar equivalent of water. One problem is that the catalyst used to facilitate such a reaction will also catalyze the cyclization of diethylenetriamine to form piperazine and an equivalent of ammonia. Whether the reaction mixture will contain a high

ratio of noncyclized polyalkylene polyamines to cyclized polyalkylene polyamines is a function somewhat of the reaction rates of forming a noncyclized polyalkylene polyamines and the reaction rates of forming cyclized polyalkylene polyamines from the noncyclized polyalkylene polyamines.

The reaction is preferably conducted in the vapor phase or the supercritical phase, but can be conducted in the liquid phase. For purposes herein, sometimes, the term gas phase encompasses both the vapor phase and supercritical phase. The reaction is usually conducted at a temperature between 125° and 425°C., preferably between 220° and 350°C., and most preferably between 260° and 300°C. The reaction is usually conducted at a pressure of about 50 to 4,000 psig, preferably greater than 100 psig and most preferably between 200 and 2,000 psig. The exact pressure used of course depends upon the desired mole ratio of noncyclics to cyclics. Increasing the pressure raises the ratio.

Decreasing the pressure lowers the ratio. The liquid hourly space velocity of the reactants in the reaction is between about 0.1 and about 100 cubic feet per hour and preferably between about 1 and about 25 per hour.

Ammonia can be used in the reaction. A stoichiometric excessive amount of ammonia can be used as a gaseous compatible adjuvant in the reactor. The supercritical temperature of ammonia is 130°C. -at and above such temperature, additional pressure will not cause the ammonia to go back into the liquid phase.

Preferably the mole ratio of alkyleneamine to alkanolamine in the reaction zone is from 4:1 to 1:4.

In a preferred embodiment of the invention process, preferably the alkylene amine compound is ethylenediamine, the alkanolamine compound is monoethanolamine, the molar ratio of ethylenediamine to monoethanolamine is greater than 1 to 1, the gaseous, the gaseous compatible adjuvant is ammonia, the molar ratio of ammonia to ethanolamine is less than 10 to 1, and the pressure during the reaction is raised or lowered in order to control the mole ratio of noncyclic products to cyclic products as desired.

The phosphorus-containing catalyst is advantageously a solid, insoluble, metal acid phosphate. One group of preferred catalysts are the Group IIIB metal phosphates, Group IIIB metal monohydrogen phosphates and Group IIIB metal dihydrogen phosphates supported on an inert carrier, such as, alumina, silica, silica-alumina, diatomaceous earth or silica-titania. Somewhat higher reaction temperatures are used with the Group IIIB metal acid phosphate catalysts.

The invention further involves increasing the pressure at which the reaction is conducted as needed throughout the reaction so as to maintain the desired mole ratio of the produced noncyclic polyalkylene polyamines to the produced cyclic polyalkylene polyamines. The pressure is increased with catalyst age, i.e., as the catalyst deactivates with time.

## DETAILED DESCRIPTION OF THE INVENTION

As used herein, all parts, percentages, ratios and proportions are on a weight basis and all temperatures are in degrees Centigrade, unless otherwise stated or otherwise obvious herefrom. Also as used herein, the term "ethanolamine" specifically means monoethanolamine unless otherwise indicated or implied herein. U. S. Sieve Series mesh sizes are used herein.

The alkyleneamine having at least two amino groups is preferably an unbranched alkylene moiety, such as, ethylenediamine, and preferably has primary amino groups. The alkanolamine preferably has a primary or secondary hydroxy moiety and preferably amino group(s). Preferably, the alkanolamine has an unbranched alkylene moiety.

The alkanolamine compounds used in the invention process include those represented by the formula:

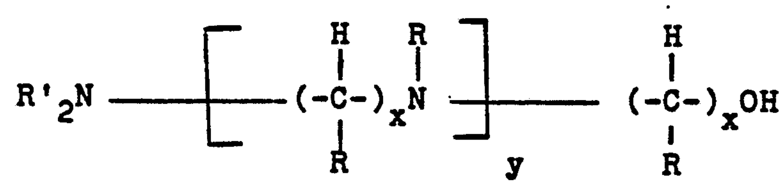

wherein R is hydrogen or a lower alkyl ($C_1$ to $C_4$) radical, R' is hydrogen or an alkyl ($C_1$ to $C_{25}$) x is a number from 2 to 6, and y is a number from 0 to 3. Examples of suitable alkyl radicals are the lower -($C_1$ to $C_4$) alkyls, such as, methyl, ethyl and butyl, and higher alkyls, such as, octyl, decyl and octadecyl. Methyl is the preferred lower alkyl radical. However, it is preferred that R and R' both are hydrogen; thus the alkanolamine would contain a primary amino group. Examples of useful alkanolamine compounds are the ethanolamines, isomeric propanolamines, N-(2-aminoethyl) ethanolamine, N-methylethanolamine, N,N-dimethylethanolamine, N,N,N'-trimethylaminoethylethanolamine and the like. Recycle of at least a portion of the unreacted alkanolamine recovered from the product stream to the reactor allows control of the molecular weight distribution and range of the produced polyalkylene polyamines.

The alkyleneamine reactants used in the invention process are represented by the formula:

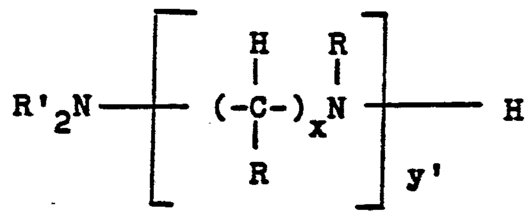

wherein R is hydrogen or a lower alkyl ($C_1$ to $C_4$) radical, R' is hydrogen or an alkyl ($C_1$ to $C_{25}$) radical, x' is a number from 2 to 6, and y' is a number from 1 to 4. Examples of suitable alkyl radicals are the lower ($C_1$ to $C_4$) alkyls, such as, methyl, ethyl and butyl, and higher alkyls, such as, octyl, decyl and octadecyl. It is preferred that R and R' are both hydrogen. The preferred lower alkyl radical is methyl. Examples of useful alkyleneamine compounds are 1,3-propylenediamine, N-methylpropylenediamine, 1,2-propylenediamine, diethylenetriamine, tributylenetetraamine, triethylenetetraamine, N,N,N'-trimethyldiethylenetriamine, noncyclic isomers of triethylenetetramine, noncyclic isomers of tetraethylenepentamine, N-methylethylenediamine, N,N-dimethylethylenediamine and ethylenediamine, which is the preferred alkyleneamine compound. Recycle of at least a portion of the alkyleneamine separated from the product stream to the reactor allows control of the molecular weight distribution and range of the produced polyalkylene polyamines.

Generally, the mole ratio of alkyleneamine compound to alkanolamine compound can range from about 0.05:1 to 12:1, preferably about 4:1 to 1:4, and most preferably is about 1:1.

When a phosphorus-containing catalyst, particularly a metal acid phosphate catalyst, is used, noncyclic polyalkylene polyamines are generally predominantly produced. This tendency is overcome by many factors, such as, long residence time in the reaction zone usually causes the relatively amount of cyclics to rapidly increase. The invention allows control of the mole ratio of noncyclics to cyclics to be controlled regardless of such multiple reaction factors.

Noncyclic polyalkylene polyamines that are generally produced by the reaction of alkanolamine (plus an alkyleneamine) are represented by the formula:

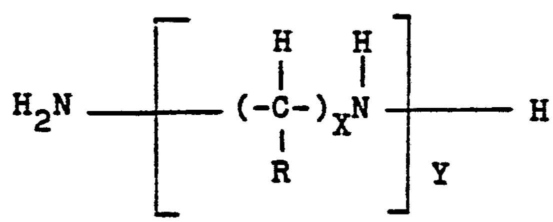

wherein R is hydrogen or a lower alkyl ($C_1$ to $C_4$) radical, preferably a methyl radical, X is a number from 2 to 6, Y is a number from 2 to 7, and X can vary for a given value of Y. Examples of such noncyclic polyalkylene polyamines that are produced are dipropylenetriamine, tributylenetetramine, di(2-methylethylene)triamine, tri (2-methylethylene)tetramine, N-(2-aminoethyl)-1,3-propylenediamine, diethylenetriamine, the noncyclic isomers of triethylenetetramine and the noncyclic isomers of tetraethylenepentamine. It is usually desirable not to coproduce significant amounts of by-products, particularly cyclic amines, especially piperazine and aminoethylpiperazine, that are

often of less commercial value than diethylenetriamine and other noncyclic polyalkylene polyamines.

Noncyclic polyalkylene polyamines include polyalkylene polyamines having straight-chain and/or branched alkylene groups.

Cyclic polyalkylene polyamines that are produced by the reaction of (a) an alkyleneamine and an alkanolamine or (b) ammonia, an alkyleneamine and an alkanolamine or (c) an alkanolamine and ammonia are represented, for example by the following formula:

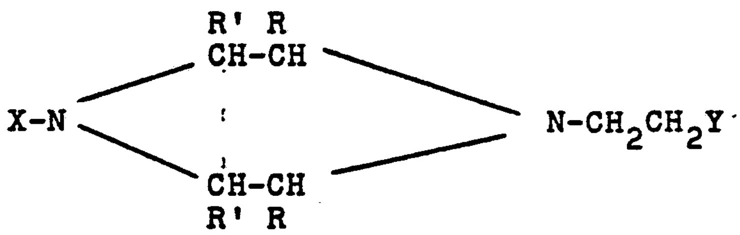

wherein R is hydrogen, an alkyl group containing 1 to 12 carbon atoms or a cycloakyl group containing 6 to 12 carbon atoms, R' is hydrogen or an alkyl group containing 1 to 4 carbon atoms, X is hydrogen or $-CH_2CH_2Y$ and Y is -OH or $-NH_2$.

Examples of cyclic polyalkylene polyamines are piperazine, N-(2-hydroxyethyl)piperazine, N,N-di(hydroxyethyl)piperazine, N-(hydroxyethyl)-2-methylpiperazine, N,N,'-di(hydroxyethyl-2-methylpiperazine, N-(hydroxyethyl)-2-ethylpiperazine, N,N'-di(hydroxyethyl)-2-ethylpiperazine, N-(hydroxyethyl)-2-butylpiperazine, N,N-di-(hydroxyethyl)-2-butylpiperazine, N-(hydroxyethyl)-2-dodecylpiperazine, N-(hydroxyethyl)-2-cyclohexylpiperazine, N-(hydroxy ethyl)-2-hexylcyclohexylpiperazine, N,N'-(dihydroxyethyl)-2,5-dimethylpiperazine, N-(hydroxyethyl)-2,3,5,6-tetramethylpiperazine, N,N'-di(hyroxyethyl)-2,5-dimethylpiperazine, N-(hydroxyethyl)-2,5-diethylpiperazine, N-(hydroxyethyl)diethylenetriamine, N-(hydroxypropyl)diethylenetriamine, N-(2-hydroxybutyl)dipropylenetriamine and morpholine.

The phrase "predominately noncyclic polyalkylene polyamines "is meant to mean greater than about 50 weight percent of linear and branched polyalkylene polyamines in the total polyamine product.

In the reactor, the temperature for the reaction depends upon the particular starting materials, ratios of reactants, and most importantly, the activity of the catalyst used. Generally, in the process of the invention, temperatures within the range of 125° to 425°C. are suitable while a preferred range is 220° to 350°C. and the most preferred range is 260 to 300°C. Manipulation of the reaction temperature can be used to raise or lower the reaction pressure. A gaseous compatible adjuvant can be utilized.

The pressure at the time of reaction should normally be within the range from about 50 to about 4,000 psig, preferably greater than 100 psig and most preferably from 200 to 2,000 psig. Normally, a gaseous compatible adjuvant, such as, helium, methane, water, hydrogen, nitrogen or argon, can be added to increase the pressure in a batch reactor and volumetric flow in a fixed bed reactor. The use of pressure in the reaction zone to control the mole ratio of the produced noncyclic polyalkylene polyamines to the produced cyclic polyalkylene polyamines is explained in detail below.

The reaction is preferably conducted in the vapor phase or the supercritical phase. As sometimes used herein the phrase "gas phase" mean vapor phase and supercritical phase. The liquid hourly space velocity of the reactants (EDA and MEA or EDA, MEA and $NH_3$) is between about 0.1 and about 100 per hour and preferably between about 1 and about 25 per hour.

By reaction zone is meant that vessel, e.g., autoclave, continuous stirred tank reactor or packed bed reactor, in which the catalyst is located and production of polyalkylene polyamines is effected.

Although the reactions can be carried out in the batch mode, they are preferably conducted as continuous processes, for example, operation of a continuously stirred tank reactor or a packed (fixed) bed reactor. The continous process is carried out by employing conventional process techniques and apparatus well known to those skilled in the art. In the continuous reaction processes, the catalyst can be added alone or in combination with the reactants, or, as stated above, the catalyst can be provided as a fixed bed on conventional support materials well known to those skilled in the art. The reaction is allowed to proceed until a desired conversion is obtained or the reaction is complete.

Normally the reaction is carried out within about 0.5 to 5 hours in the batch mode or residence times (based on the alkanolamine and alkyleneamine components) of 0.1 to 4.0 hours in a continuous mode for practical levels of polyalkylene polyamine production.

The reactor can be an up-flow or down-flow reactor and can have a fluidized bed or, most commonly, a fixed bed. The catalyst bed can contain inert particles which can be interspersed throughout the bed and/or form discrete layers, e.g., at an end or intermediary to the bed. The volume of the reaction zone containing such inert particles is the reaction zone volume for purposes of determining the feed rate. Preferably, the space velocity should not be so high that for the reactor geometry, a significant amount of backmixing occurs. Advantageously, the flow through the catalyst bed is substantially plug-type flow.

Ammonia or a primary amine or a secondary amine can also be used in the feed to the reactor. The ammonia is both a reactant and a diluent - (when excess of it is present) in the invention process. When ammonia is used, the mole ratio of ammonia (or the primary amine or the secondary amine) to the alkyleneamine and alkanolamine is usually about 20:1 to 0.6:20 and preferably is in the range of 12:1.25 to 1:1. Ammonia and the preferred primary and secondary amines which are used in the invention process are represented by the formula:

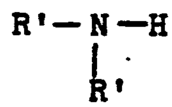

wherein R' is hydrogen or an alkyl ($C_1$ to $C_{25}$) radical, preferably a lower alkyl ($C_1$ to $C_4$) radical, such as methyl or ethyl. Stoichiometrically, one molecular unit of ammonia or amine (primary or secondary) is required per molecular unit of hydroxyl group. However, the formation of diethylenetriamine is favored by the presence of excesses of ammonia, but a practical limit on the amount of ammonia employed exists due to energy consumption. Useful amine feedstocks include monomethylamine, dimethylamine, monoethylamine, diethylamine, octylamine and octadecylamine.

Use of secondary amines instead of ammonia leads to polyalkylene polyamines containing terminal dialkylamino groups. Alternatively, use of primary amines instead of ammonia leads to polyamines which contain randomly distributed monoalkylamino groups.

The catalysts used in the invention are heterogeneous catalysts. The catalysts are used in an amount of 0.1 to 12 weight percent, preferably 0.5 to 10 weight percent, and most preferably 2 to 7 weight percent, based on the total weight of the reactants.

Any phosphorus-containing catalyst can be used. One group of preferred phosphorus-containing catalysts are the metal phosphate catalysts, which can be metal phosphates, metal monohydrogen phosphates, metal dihydrogen phosphates and metal pyrophosphates.

The metal phosphate catalysts include boron phosphate, aluminum phosphate, ferric phosphate, zinc phosphate, ferrous phosphate, nickel phosphate, chromium phosphate, copper phosphate and cobalt phosphate. Other metal phosphate catalysts which can be used are the phosphate of lithium, sodium, potassium, other metals of Group IA of the periodic table , beryllium, magnesium, calcium, other metals of Group IIA of the periodic table, titanium, zirconium, other metals of Group IVB of the periodic table antimony and tin (valence states II and IV). Further useful ctalysts are those which comprise a phosphorus bonded to a Group IVB transition metal oxide support, such as are disclosed in published European Patent Application 0115138 (the pertinent parts of which are incorporated herein by reference). Mixtures of two or more of the metal phosphate catalysts can be used.

The metal phosphate catalysts also include the pyrophosphates, monohydrogen phosphates and dihydrogen phosphates of strontium, copper, magnesium, calcium, barium, zinc, aluminum, cobalt, nickel, cerium, neodymium, and mixtures thereof. Specific examples of such catalysts are $SrHPO_4$, $Sr/BaHPO_4$, $Sr(H_2PO_4)_2$, $Ca(H_2PO_4)_2$, $Nd_2(HPO_4)_3$, $Ce_2(HPO_4)_3$, $CoHPO_4$, $NiHPO_4$, $A1_2(HPO_4)_3$, $MgHPO_4$, $BaHPO_4$, $CuPHO_4$ and $ZnHPO_4$.

The metal phosphate catalysts include crystalline zirconium phosphate of U. S. Patent No. 3,416,884 and granular zirconium phosphate of U. S. Patent No. 4,025,608 -the pertinent parts of such patents are incorporated herein by reference.

The metal phosphate catalysts which are most preferred for practicing the process of the invention are Group IIIB metal acid phosphates including Group IIIB metal phosphates, Group IIIB metal monohydrogen phosphates, Group III metal dihydrogen phosphates and mixtures thereof. U. S. Patent No. 4,463,193 (the pertinent parts of the patent are incorporated herein be reference) discloses processes for preparing the Group IIIB metal acid phosphates. While the intent of the catalyst preparation is to specifically provide a particular Group IIIB monohydrogen phosphate or dihydrogen phosphate, mixtures of the Group IIIB metal phosphates of the above-mentioned types may be obtained owing to complicated dependence of the catalyst composition on preparation conditions. Nevertheless, although the Group IIIB metal acid phosphate catalyst of the invention comprises the metal phosphate, monohydrogen phosphate, dihydrogen phosphate or mixtures thereof, the monhydrogen and dihydrogen phosphates of the Group IIIB metals are the preferred catalysts when in relatively pure form individually or in combination.

A Group IIIB metal is meant to include scandium, yttrium, lanthanum and the rear earth lanthanide metals having atomic numbers 58 to 71, and the rare earth actinides having atomic numbers 89 to 92.

The most preferred metal phosphate catalysts for the production of noncyclic polyalkylene polyamines are the acid phosphates, preferably the monohydrogen phosphates and dihydrogen phosphates, of scandium, lanthanum, cerium, samarium, europium, thulium, erbium, ytterbium, yttrium, leutium, thorium, neodymium, praseodymium, dysprosium and gadolinium.

The acid phosphate catalysts can be used for the production of polyalkylene polyamines either singly or in combination.

Examples of the most preferred catalyst compounds include lanthanum monohydrogen phosphate, lanthanum dihydrogen phosphate, lanthanum phosphate, praseodymium dihydrogen phosphate, praseodymium phosphates, neodymium monohydrogen phosphate, neodymium dihydrogen phosphate, neodymium dihydrogen phosphate, neodymium phosphate, and mixtures thereof.

The quantity of the acid phosphate salts of the Group IIIB metals used in the reaction can vary widely depending upon the reactivity of the catalysts and the reactivity of the reactants present. A catalytically effective amount of material is used; in other words, an amount which causes a reaction, the alkyleneamine compound and the alkanolamine compound to yield polyalkylene polyamine products at the temperature and pressure used. Usually though, the amount used to provide a catalytic effect ranges from about 0.1 to 25 mole percent based upon the total amount of alkyleneamine compound and alkanolamine compound feed present in the reaction mixture, and preferably is an amount of about 0.1 to 10 mole percent. Within these ranges though, the level of catalyst is empirical and is adjusted depending on the product slate desired.

The Group IIIB metal phosphate catalysts used in the process of the invention can be prepared by the precipitation of the desired metal acid phosphate salt, washing the salt to remove inorganic coproducts and drying the salt. Optionally, dried catalysts can be further processed prior to use for polyalkylene polyamine manufacture.

Such processing is well known to those skilled in the art and includes extrusion or pelletizing or compounding with an inert support such as alpha-alumina.

Methods of preparing Group IIIB metal monohydrogen phosphate or dihydrogen phosphate are disclosed in U. S. Patent No. 4,324,917 (the pertinent parts of the patent are incorporated herein by reference). Phosphate-containing materials can be obtained which consists predominantly of the Group IIIB metal phosphate, the Group IIIB metal monohydrogen phosphate, the Group IIIB metal dihydrogen phosphate, or mixtures in varying proportions of the Group IIIB metal monohydrogen and dihydrogen phosphate, and/or mixtures in varying proportions of any of the above Group IIIB metal acid phosphates with the Group IIIB metal phosphate. Such variations in catalyst composition can result from dependence of the catalyst composition on preparation conditions, such as temperature, concentration of reagents, stoichiometry of reagents, rate and order of reagent additions, pH of preparation, duration of preparation, volume and pH of water wash, duration of catalyst washing, and duration and temperature of catalyst drying. In any event, the Group IIIB metal acid phosphates obtained according to the general preparations referred to above are catalytically active for the production of polyalkylene polyamines.

Published European Patent Application 0115138 (the pertinent parts of which are incorporated herein by reference) discloses methods for preparing the catalysts comprising a phosphorus bonded to a Group IVB metal oxide support. Any appropriate liquid or liquefiable phosphorus compound can be used as a source of the phosphorus. For convenience, phosphoric acid will normally be used. However, other phosphorus compounds such as phosphoryl chloride ($POCl_3$), phosphorus acid,

polyphosphoric acid, phosphorus halides, such as phosphorus bromide, alkyl phosphates and alkyl phosphites such as trimethyl phosphate, triethyl phosphate, trimethyl phosphite, triethyl phosphite, etc. may be utilized. Also, a diaminohydrogen phosphate such as diammonium hydrogen phosphate, $(NH_4)_2HPO_4$, dimethyldiamino hydrogen phosphate, diethylaminohydrogen phosphate, etc. may be used. The catalyst compositions are prepared by depositing a phosphorus compound on a support comprising an oxide of a group IVb transition metal oxide. The group IVb transition metal oxides include the oxides of titanium, zirconium, hafnium and thorium. Pellets of the group IVb metal oxide may be prepared by extrusion or by compaction in conventional pelleting apparatus using a pelleting aid such as graphite. The phosphorus compound can be on a powdered IVb metal oxide followed by pelleting and calcination. Preferably the catalyst composition is prepared by impregnating a preformed pellet. A suitable procedure to be used is to heat a liquid containing the liquid or liquefiable phosphorus compound at a temperature of about 100° to about 150°C. and to then add pellets in an amount about equal to the volume of the heated liquid. This treatment should be continued from about 0.5 to about 5 hours. At the end of that time, the resulting mixture of pellets and liquid is cooled, decanted to remove excess liquid followed by washing with an amount of water adequate to substantially completely remove unadsorbed liquid. Temperatures above 150°C. can be used, if desired, but there is no particular advantage in doing so. It will be understood that the phosphorus that is present on a thus-treated pellet is not present as elemental phosphorus, but rather as phosphorus that is chemically bound, probably as an oxide, to the group IVB metal oxide support. However, the exact nature of the bonding is not completely understood.

European Patent Application 0115138 discloses that the amount of phosphorus that is bonded or otherwise adheres to the support is a function of heating and other conditions used in the treating step and is also a function of the chemical identity of the phosphorus compound that is used as a source of phosphorus. Under the treating conditions exemplified above, at least about 2.5 weight percent of phosphorus is caused to bond or otherwise permanently adhere to the pellets. There is an upper limit to the amount of phosphorus that bonds or otherwise permanently adheres to the support. This upper limit is, as indicated, a function of both the treating conditions and the chemical used as a source of the phosphorus. Normally, the maximum amount of phosphorus that can be caused to bond or otherwise permanently adhere to the pellets is within the range of about 5 to 10 weight percent. As a matter of convenience, the normal practice is to use only one chemical as a phosphorus source - (e.g., phosphoric acid). However, mixtures of two or more such reagents may be used, if desired. Calcining is not mandatory if the pellets are impregnated at least at about 100°C.,but the pellets can be calcined, if desired. Calcining is conducted for 2 to 24 hours at a temperature of 100°C or above but below the temperature at which thermal destruction of the phosphorus bonding occurs. This can be determined experimentally for a particular catalyst. Temperatures above 900°C. should be avoided. A suitable calcining temperature range is normally 200° to 800°C., and, more preferably 500° to 700°C. Other procedures can be used in adding phosphorus to the group IVb metal oxide.

The pertinent parts of copending, commonly-assigned U. S. Application Serial No. 576,807, filed on February 7, 1984, are incorporated herein by reference. U. S. Ser. No. 576,807 discloses certain phosphorus acid or acid derivative compounds which are useful phosphorus-containing catalysts within the scope of the invention herein. The term phosphorus acid or acid derivative defines compounds having a P-X wherein P is a phosphorus atom bonded to a halogen, oxygen, sulfur or nitrogen atom is X which is a radical capable of (1) hydrolyzing to produce the corresponding phosphorus acid structure, or (2) exchanging with a hydroxyl group from the hydroxy alkylene reactant to provide a phosphorus ester.

The phosphorus acid or acid derivative catalyst of U. S. Ser. No. 576,807 is believed to function by forming with the alkanolamine or alkylene glycol compound a phosphorus ester in situ. For this reason, it is believed that a requirement for a good phosphorus catalyst is that it contain as a substructure an atom bonded to phosphorus that can be replaced readily by the oxygen atom of a hydroxyl group of the difunctional hydroxy alkylene compound. Such a replaceable atom might be oxygen (as in the case of phosphorous or phosphoric acid or their esters), halogen, nitrogen (as in the case of amides of phosphorous or phosphoric acids) or another atom that can be transformed into a phosphorus ester by a similar process.

Phosphorus-containing compounds such as trialkyl and triaryl phosphines and phospine oxides, which contain no such exchangeable substructure, do not function as catalysts as defined in U. S. Ser. No. 576,807. Very sterically hindered phosphorus compounds such as hexaethyl phosphoric triamide, while containing the requisite exchangeable substructure and functionality to some extent, are less

preferred catalysts because they undergo the exchange process with the alkanolamine or alkylene glycol hydroxyl moieties only slowly. Phosphorus acids are defined by those structures wherein X in the P-X radial is a hydroxyl radical. Acid derivatives are defined by structures wherein X is a substitute functional group. Various acids derivatives include: salts when -X is $-O^-M^+$ wherein $M^+$ is a mono or polyvalent cation; amides when -X is bonded to the phosphorus atom through a nitorgen atom; anhydrides when -X contains a second phosphorus atom bonded to the first phosphorus atom through an oxygen atom; esters when -X is -OR; and so on with regard to other functional groups defined by -X. The precise phosphorus acid or acid derivative structure is not critical so long as it fulfills the following two functional requirements: (1) that it provides for the relatively selective production of predominantly linearly extended polyalkylene polyamines and (2) that it enables increased conversion rates for polyalkylene polyamine production when water is removed during the reaction, possibly due to the water-inhibited formation of a phosphorus intermediate compound during the reaction.

The phosphorus acids or acid derivative catalysts of U. S. Ser. No. 576,807 include those having the structure:

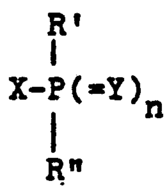

wherein Y is an oxygen or sulfur atom; n is 0 or 1; X is hydroxy, alkyoxy, aryloxy, or the thio analogs of the foregoing, alkyl or aryl substituted amino, halo, or the salts or phosphorus anhydrides or thioanhydrides of the foregoing when X is hydroxy or mercapto; R' and R" are hydrogen, alkyl, aryl or one of the groups previously defined by X.

Suitable phosphorus acid or acid derivatives of U.S. Ser. No. 576,807 which can be employed include, for example, acidic metal or semi-metal phosphates, phosphoric acid compounds, and their anhydrides, phosphorous acid compounds and anhydrides, alkyl or aryl phosphates, alkyl or aryl phosphites, alkyl or aryl substituted phosphonic acids and phosphinic acids, alkali metal monosalts or phosphoric acid, phosphorous amides and phosphoric amides, the thioanalogs of the foregoing, and mixtures of any of the above. Suitable acidic metal or semi-metal phosphates include boron phosphate, ferric phosphate, aluminum phosphate and the like. Suitable phosphoric acid compounds include aqueous or anhydrous phosphoric acids, such as orthophosphoric acid, pyrophosphoric acid, metaphosphoric acid, and condensed phosphoric acids such as polyphosphoric acids. Any commercially available mono-, di-, or trialkyl or aryl phosphate or phosphate ester can be employed. In addition, bis-(phosphates) and secondary phosphate esters, such as those disclosed in U. S. Patent No. 3,869,526 and U. S. Patent No. 3,869,527, respectively, can be utilized. Suitable alkyl or aryl substituted phosphonic acids or phosphinic acids include alkyl phosphonic acids, aryl phosphonic acids, alkyl phosphinic acids and aryl phosphinic acids. Examples of such phosphorus acid or acid derivative compounds include phenylphosphinic, ethylphosphonic, phenylphosphonic, naphthaphosphonic, and methylphosphinic acids; methyl phenylphosphonate, dimethyl phenylphosphonate, methyl phenylphosphinate, ethyl naphthaphosphinate, propyl methylphosphonate; hexamethyl phosphoric triamide, hexaethyl phosphoric triamide and their analogous phosphorous triamides. Preferred phosphorus catalysts include hexamethyl phosphorous triamide, hexaethyl phosphorous triamide, boron phosphate, ferric phosphate, aluminum phosphate, phosphoric acid and phosphorous acid.

The amount of phosphorous acid or acid derivative catalyst of U. S. Ser. No. 576,807 utilized is a catalytically effective amount to cause condensation of the reactants to produce predominantly diethylenetriamine. This quantity will vary depending upon the reaction conditions and catalyst utilized. Usually a catalytically effective amount will be from about 0.01 to about 10 mole percent and preferably from about 1 to about 3 mole percent, based on the moles of hydroxyl alkylene compounds used.

The pertinent parts of copending, common-assigned U. S. Application Serial No. 606,000, filed on May 2, 1984, are incorporated herein by reference. U. S. Ser. No. 606,000 discloses certain phosphorus amide catalysts whic are compounds

having at least one phosphorus-nitrogen, i.e., P-N, bond. Preferably, the P-N bond is part of a -P-N-H or P-N-C-substructure. Compounds containing suitable P-N bonds can have three, four, or five substituents about the phosphorus.

Suitable compounds catalysts of U. S. Ser. No. 606,000 having three substituents about phosphorus can be defined by the formula:

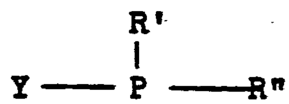

wherein Y is an unsubstituted or alkyl and/or aryl substituted amino radical: R' and R" are hydroxy, alkoxy, aryloxy, or their thio analogs, hydrogen, alkyl, aryl, hale, or one of the groups previously defined by Y, and can be joined together with each other or with Y to form a phosphorus-containing heterocyclic ring. If R', R", or Y contains hydrogen bonded to O, S, or N, such as when R' or R" is hydroxy or mercapto or Y is monoalkylamino, then corresponding metal salts containing P-O-M, P-S-M, or P-N-M linkages, where M is a monovalent or polyvalent metal or semimetal ion, and anhydrides, thioanhydrides, and condensed phosphorus amides containing respectively P-O-P, P-S-P, and P-N-P linkages can be suitable catalysts as well.

Suitable phosphorus amide catalysts of U. S. Ser. No. 606,000 having four substituents about phosphorus include those having the formula:

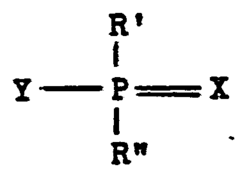

wherein X is an oxygen or sulfur atom, preferably oxygen, and Y, R', and R" are as defined above. As previously, corresponding metal and semimetal salts and condensed phosphorus compounds may also be suitable.

Suitable phosphorus amide catalysts of U. S. Ser. No. 606,000 having five substituents about phosphorus include those having the formula:

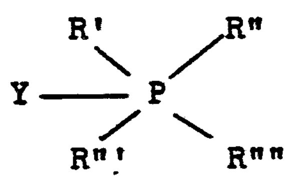

wherein Y is defined as above and R', R", R"', and R"" are as defined for R' and R" above. As previously, corresponding metal and semimetal salts and condensed phosphorus compounds may also be suitable.

Suitable phosphorus amide compounds which can be employed include, for example, the following compounds or their alkyl or aryl derivatives:

| | |
|---|---|
| phosphoramidous acid, | $N_2N-P(OH)_2$; |
| phosphordiamidous acid, | $(H_2N)_2POH$; |
| phosphordiamidic acid, | $(H_2N)_2P(O)(OH)$; |
| phosphoramidic acid, | $H_2NP(O)(OH)_2$; |
| alkyl and aryl phosphonamidic acids, | $RP(O)(OH)NH_2$; |
| alkyl and aryl phosphonamidous acids, | $RP(OH)NH_2$; |
| esters and half-esters of the foregoing | e.g. $H_2NP(OEt)_2$; |
| metal salts of the foregoing, | e.g. $H_2NP(O)_2K_2$; |
| triaminophosphine, | $(H_2N)_3P$; |
| triaminophosphine oxide | $(H_2N)_3P(O)$; |
| alkyl and aryl phosphonic diamides, | $RP(O)(NH_2)_2$; |
| alkyl and aryl phosphonous diamides | $RP(NH_2)_2$; |
| alkyl and aryl phosphinous amides, | $R_2P(NH_2)$; |
| alkyl and aryl phosphinic amides, | $R_2P(O)(NH_2)$; |
| analogs of the foregoing substituted with alkyl or aryl groups on nitrogen, | e.g. $R_2NP(OH)_2$; |
| and thianalogs of the foregoing, | e.g. $R_2NP(S)(OEt)_2$. |

The alkyl or aryl substituents on these substances can be linked to phosphorus through more than one atom, so as to form cyclic members of the above classes containing such heterocyclic rings as 1,3,2-diazophospholidine,

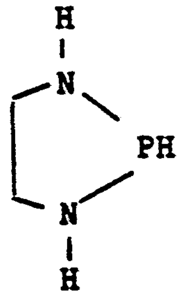

; 1,3,2-oxazaphospholidine,

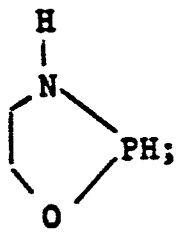

tetrahydro-2H-1,3,2-oxaza phosphorine,

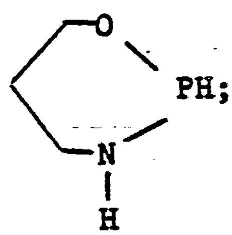

and the like. Such cyclic phosphorus amides can also be used as catalysts in the invention.

An additional class of phosphorus amides of U.S. Ser. No. 606,000 that can be useful as catalysts in the invention comprising azophosphoranes in which nitrogen is bound directly to phosphorus. Examples of such compounds include: 1,6-dioxa-4,9-diaza-5-phospha-(5-$P^V$)spiro [4.4]=nonane, H

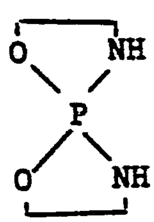

and 2,3,5,6,8,8-hexahydro-8-methyl-[1,3,2]-oxazaphospholo-[2,3-b][1,3,2]oxazaphosphole,

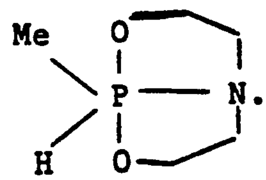

and

Preferred phosphorus amide catalysts of U. S. Ser. No. 606,000 include hexamethyl phosphorous triamide, hexaethyl phosphorous triamide and the phosphorus amide reaction product of ethylenediamine with phosphoric or phosphorous acid.

Phosphorus-containing cation exchange resins can be used in this invention and can be prepared by the methods disclosed in U. S. Patent No. 4,324,917. The cation exchange resins provide exchangeable phosphorus-containing ions such as phosphonous, phosphonic, phosphoric and phosphorous. Preferably the resins useful here are weak-acid cation exchange resins contianing one or more of the above phosphorus-containing exchangeable ions. Duolite® resins available from Diamond Shamrock Corp. are typical commercial phosphorus-containing resins. Examples of these are Duolite® ES-62, Duolite® ES-63, and Duolite® ES-65 which are the phosphonous, phosphonic and phosphoric acid types, respectively.

Any of the non-resin catalysts useful in the invention can be supported on carriers, such as, silica, silica-alumina, silica-titania, alumina, diatomaceous earth (Kieselguhr) and any other conventionally-employed inert reactor packing material. Generally, the catalysts are supported. The active catalyst species are provided on the surface of the support through, for example, coating or impregnation. The catalyst (say metal) components on the support often comprise about 1 to 50, say, about 3 to 30, weight percent of the catalyst. Useful supports can be porous and have surface areas of from about 0.1 to 500, say, about 0.3 to 100 square meters per gram.

The catalyst can be of any convenient size or shape. Catalysts can be made in the form of powders, spherical or conical pellets, extruded strips and the like. Impregnated spherical pellets ranging in diameter from 1/8 inch to 3/16 inch and extruded strips of a cylindrical-type shape ranging from 1/32 inch to 1/2 inch in length are typical of those which can be used as supports. Often, for commercial-scale operations, the pellets range in diameter from about 0.1 to 1 centimeter.

Recovery of the polyalkylene polyamines, alkanolamine and alkylenediamine from the reaction mixture from the reactor can be accomplished by conventional techniques.

Preferably the separation step is conducted using distillation. Most preferably the separation is conducted using a fractional distillation column with the polyethylene polyamines coming off of the bottom of the column and with the unreacted alkanolamine and alkylenediamine coming off of the top portion of the column. With a fractional distillation column, for example, having 10 trays, a pressure of 1000 psig and a base temperature of 220°C., 90 mole percent of the unreacted ethanolamine and ethylenediamine come off of the top of the column with the unreacted $NH_3$, and 90 mole percent of the diethylenetriamine comes off of the bottom of the column.

The separation can be conducted in absorbers, with the absorbing liquids removing the polyalkylene polyamines from the unreacted alkanolamine and alkyleneamine. Preferably the absorbing liquid for the polyalkylene polyamines is triethylenetetramine or a higher boiling adsorbent.

The separation can also be conducted using a series of partial condensers. The alkanolamine and alkyleneamine flash from the first of the partial condensers, and the polyalkylene polyamines condense out in the first of the partial condensers. Preferably 3 to 5 partial condensers are used.

The analysis of the products produced by the process of the invention can be conducted by using standard gas chromatography techniques using columns selected from their ability to separate the individual components that may be present in a particular reaction mixture.

Polyethylene polyamines are useful as corrosion inhibitors, fabric softeners, lubricating oil additives, co-monomers for polyamide resins, fungicides, surfactants, curing agents for epoxy resins and chelating agents.

Copending, commonly-assigned patent application entitled "Interreactor Separator", (D-14871), concurrently filed with this application, is incorporated herein by reference. Such application discloses the use of a reductive amination reaction zone from which its effluent is separated into a,

preferably, gaseous, MEA-and EDA-containing phase and a liquid, DETA-rich phase. The gas phase can be used for recycle (advantageously, it is at high pressure and suitable for recycle without undue energy penalties) or as a feed to another reactor which can use a reductive amination or other type (e.g., phosphorous-based catalyst) of catalyst.

Copending, commonly-assigned patent application entitled "Improving The Quality Of Catalytically Prepared Polyalkylene Polyamines", (D-14872), concurrently filed with this application, is incorporated herein by reference. Such application discloses processes for making polyalkylene polyamines from ethanolamines and a nitrogen compound (e.g., ammonia or alkyleneamine) using a phosphorous-based catalyst in the presence of sufficient hydrogen to enhance color and reduce odor.

Copending, commonly-assigned patent application entitled "Conversion Of Oxygen-Containing Polyamines", (D-14873), concurrently filed with this application, is incorporated herein by reference. Such application discloses, in its broadest aspect, passing an oxygenated, polyethylenepolyamine feed in the presence of a nitrogen compound to a reaction zone containing a phosphorous-based catalyst. Advantageously, this feed is obtained from a polyethylenepolyamine reactor having a reductive amination catalyst. The feed typically contains AEEA.

Copending, commonly-assigned patent application entitled "Two Reactor Scheme Using Only Phosphorus-Containing Catalysts", (D-14874) concurrently filed with this application, is incorporated herein by reference. Such application discloses passing an oxygenated, polyethylenepolyamine feed in the presence of a nitrogen compound to a reaction zone containing a phsophorous-based catalyst. This feed is obtained from a polyethylenepolyamine reactor having a phosphorous-based catalyst. The feed typically contains AEEA.

Copending, commonly-assigned patent application entitled "Interchangeable Process Scheme", (D-14875), concurrently filed wth this application, is incorporated herein by reference. Such application discloses processes for making and separating ethyleneamines, alkylamines, morpholine, etc., in the same process equipment. The separation systems required for these processes are very similar. Hence, block operation in the same reactor and separation equipment is achieved.

Copending, commonly-assigned patent application entitled "Preparation Of Diethylene Triamine With Azeotrope Recycle", (D-14876), concurrently filed with this application, is incorporated herein by reference. Such application discloses processes for making ethyleneamines using a reductive amination of phosphorous-based catalyst in which a gaseous EDA/water phase is separated from the reactor effluent and at least a portion of the separated phase is returned to the reactor. In a preferred embodiment using a reductive amination catalyst, the recycle stream is admixed with MEA to break the azeotrope with water condensing out.

Copending, commonly-assigned patent application entitled "Water Addition To Enhance Phosphoric Acid Salt Catalyst Activity", (D-14877), concurrently filed with this application, is incorporated herein by reference. Such application discloses providing beneficial amounts of water in the reaction zone in which alkanolamine and another nitrogen compound are reacted to produce alkyleneamines over a phosphorous-based catalyst. The water is believed to enhance or maintain or generate catalytic activity.

The following compound abbreviations apply herein:

EDA -ethylenediamine

MEA -monoethanolamine

PIP -piperazine

AEP -aminoethylpiperazine

DETA -diethylenetriamine

TETA(NC) -triethylenetetramine (noncyclic isomers)

TETA(C) -triethylenetetramine (cyclic isomer)

TEPA(NC) -tetraethylenepentamine (noncyclic isomers)

HVY(NC) -pentaethylenehexamine and higher oligomeric polyethylene amines

EXAMPLE 1

Lanthanum acid phosphate catalyst (10 $cm^3$ of -12 to -18 mesh particles) is charged to a fixed bed tubular reactor (18 $cm^3$ total volume) and overlaid with crushed vicor (5 $cm^3$ of -12 to -18 mesh particles). The reactor is heated to 265°C. in an insulated air oven. A mixture of ethylenediamine,

ammonia and ethanolamine (mole ratio of EDA:MEA:$NH_3$ was 1:1:6) is passed over the catalyst at 400 psig at a liquid hourly space velocity of 3.5 per hour of reactants. Analysis of the cooled reaction product by gas/liquid chromatography indicates production of a high level of noncyclic polyalkylene polyamines as compared to the amount of produced cyclic polyalkylene polyamines.

EXAMPLE 2

Example 1 is repeated, except that the pressure in the reactor is increased throughout the run to 1,400 psig. Analysis of the cooled reaction product by gas/liquid chromatography indicates production of a much higher level of noncyclic polyalkylene polyamines (as compared to the amount of cyclic polyalkylene polyamines produced) than obtained in Example 1.

EXAMPLE 3

Example 1 is repeated, except that the pressure is increased halfway through the run to 1000 psig. A sample of the reaction product is taken during the first half of the run. Analysis of such sample of cooled reaction product by gas/liquid chromatography indicates production of a high level of noncyclic polyalkylene polyamines as compared to the amount of produced cyclic polyalkylene polyamines. A sample of the reaction product is taken during the second half of the run. Analysis of such sample of cooled reaction product by gas/liquid chromatography indicates production of a much lower level of noncyclic polyalkylene polyamines (as compared to the amount of cyclic polyalkylene polyamines produced) than during the first half of the run.

EXAMPLE 4

Example 1 is repeated, except that the pressure in the reactor is reduced throughout the run to 200 psig by lowering the pressure of the incoming feed of ethylenediamine and ethanolamine to the appropriate level. Analysis of the cooled gaseous reaction product by gas/liquid chromatography indicates production of a much lower level of noncyclic material (as compared to the amount of cyclic polyalkylene produced) than obtained in Example 1.

EXAMPLE 5

Lanthanum acid phosphate catalyst supported - (16 weight percent catalyst incorporation) on a low surface area macroporous inert alumina carrier (5 $cm^3$ of -12 to -18 mesh particles) is charged to a fixed-bed tubular reactor (14 $cm^3$ total volume) and overlaid with crushed vicar (5 $cm^3$ of -12 to -18 mesh particles). The reactor is heated to 255°C. A mixture of ethylenediamine, monoethanolamine and ammonia (mole ratio of EDA:MEA:$NH_3$ was 2:1:6) is passed over the catalyst at 1,400 psig at a liquid hourly space velocity of 3.5 per hour of reactants - (based on the EDA and MEA). Analysis of the cooled reaction product by gas/liquid chromatography indicates production of a high level of noncyclic polyalkylene polyamines as compared to the amount of cyclic polyalkylene polyamines produced.

EXAMPLE 6

Example 5 is repeated, except that the pressure in the reactor is decreased throughout the run to 200 psig. Analysis of the cooled reaction product by gas/liquid chromatography indicates production of a much lower level of noncyclic polyalkylene polyamines (as compared to the amount of cyclic polyalkylene polyamines produced) than obtained in Example 5.

**Claims**

1. In a process for producing polyalkylene polyamines by contacting an alkyleneamine compound having at least two amino groups and an alkanolamine having at least one amino group in a reaction zone, the improvement comprising controlling the molecular ratio of produced noncyclic polyalkylene polyamines to produced cyclic polyalkylene polyamines by controlling the pressure at which said reaction is conducted, said reaction being conducted in the presence of a catalytically effective amount of a phosphorus-containing catalyst at a temperature and pressure sufficient to form said polyalkylene polyamines.

2. The process as claimed in Claim 1 wherein said reaction is a continuous reaction.

3. The process as claimed in Claim 2 wherein the pressure is increased to between 1,000 and 1,500 psig from a lower level so as to cause an increase of said mole ratio of said produced noncyclic polyalkylene polyamines to said produced cyclic

polyalkylene polyamines.

4. The process as claimed in Claim 2 wherein the pressure is increased to greater than 1,500 psig from a lower level so as to cause an increase of said mole ratio of said produced noncyclic polyalkylene polyamines to said produced cyclic polyalkylene polyamines.

5. The process as claimed in Claim 2 wherein the pressure is decreased to between 200 and 300 psig from a higher level so as to cause a decrease of said mole ratio of said produced noncyclic polyalkylene polyamines to said produced cyclic polyalkylene polyamines.

6. The process as claimed in Claim 2 wherein said pressure is increased during said reaction so as to cause an increase of said mole ratio of said produced noncyclic polyalkylene polyamines to said produced cyclic polyalkylene polyamines.

7. The process as claimed in Claim 6 wherein the pressure during said reaction is increased by increasing the temperature of said reaction, whereby said mole ratio is increased and the conversion of said alkyleneamine compound and said alkanolamine compound is increased.

8. The process as claimed in Claim 7 wherein ammonia is also present in the feed.

9. The process as claimed in Claim 2 wherein said pressure is decreased during said reaction so as to cause a decrease of said mole ratio of said produced noncyclic polyalkylene polyamines to said produced cyclic polyalkylene polyamines.

10. The process as claimed in Claim 9 wherein the pressure during said reaction is decreased by decreasing the temperature of said reaction, whereby said mole ratio is decreased.

11. The process as claimed in Claim 10 wherein ammonia is also present in the feed.

12. The process as claimed in Claim 2 wherein said pressure of said feed is increased before said reaction is started so as to cause an increase in the level of said mole ratio of said produced noncyclic polyalkylene polyamines to said produced cyclic polyalkylene polyamines throughout said reaction, said increased level of said mole ratio being higher than it would have been without said pressure increase.

13. The process as claimed in Claim 12 wherein the pressure of said reaction is increased by increasing the temperature in said reaction zone.

14. The process as claimed in Claim 13 wherein ammonia is also present in the feed.

15. The process as claimed in Claim 2 wherein said pressure of said feed is decreased before said reaction is started so as to cause a decrease in the level of said mole ratio of said produced noncyclic polyalkylene polyamines to said produced cyclic polyalkylene polyamines throughout said reaction, said decreased level of said mole ratio being lower than it would have been without said pressure decreased.

16. The process as claimed in Claim 15 wherein the pressure during said reaction is decreased by decreasing the temperature of said reaction, whereby said mole ratio is decreased.

17. The process as claimed in Claim 16 wherein ammonia is also present in the feed.

18. The process as claimed in Claim 2 wherein said reaction is conducted in the vapor phase.

19. The process as claimed in Claim 2 wherein said reaction is conducted in the supercritical phase.

20. The process as claimed in Claim 2 wherein reaction is conducted at a temperature between 125° and 425°C.

21. The process as claimed in Claim 2 wherein said reaction is conducted at a temperature between about 260° and 300°C.

22. The process as claimed in Claim 2 wherein the feed also contains a reactive nitrogen-containing compound selected from the group consisting of ammonia, a primary amine and a secondary amine.

23.The process as claimed in Claim 22 wherein said reactive nitrogen-containing compound is ammonia and said ammonia is present in an amount in excess of the molar amount participating in said reaction.

24. The process as claimed in Claim 23 wherein the molar ratio of said alkyleneamine to said alkanolamine to ammonia is 0.75-4/1/5-12.

25. The process as claimed in Claim 2 wherein the

liquid hourly space velocity of said reactants in said reaction is between about 0.1 and about 100 per hour.

26. The process as claimed in Claim 2 wherein the liquid hourly space velocity of said reactants in said reaction is between about 1 and about 25 hour.

27. The process as claimed in Claim 2 wherein said alkanolamine is ethanolamine and said alkyleneamine ethylenediamine.

28. The process as claimed in Claim 2 wherein said alkyleneamine compound is ethylenediamine and said alkanolamine compound is monoethanolamine, wherein the molar ratio of ethylenediamine to monoethanolamine is greater than 1 to 1, wherein said gaseous compatible adjuvant is ammonia, wherein the molar ratio of ammonia to monoethanolamine is less than 10 to 1, and wherein the pressure during said reaction is raised or lowered in order to control said mole ratio of said produced noncyclic polyalkylene polyamines to said produced cyclic polyalkylene polyamines.

29. The process as claimed in Claim 2 wherein said phosphorus-containing catalyst in said reaction is a metal acid phosphate.

30. The process as claimed in Claim 29 wherein said metal acid phosphate is a solid, insoluble, metal acid phosphate.

31. The process as claimed in Claim 29 wherein said solid, insoluble, metal acid is reactivated in situ by inclusion of water in said feed.

32. The process as claimed in Claim 29 wherein said metal acid phosphate is a Group IIIB metal phosphate, a Group IIIB metal monohydrogen phosphate or a Group IIIB dihydrogen phosphate, and wherein said Group IIIB metal is scandium, yttrium, lanthanum or a rare earth lanthanide having an atomic number from 58 to 71.

33. The process as claimed in Claim 34 wherein said catalyst is a dihydrogen phosphate salt of lanthanum, praseodymium, neodymium, samarium, dysprosiium or gadolinium.

34. The process as claimed in Claim 31 wherein said catalyst is supported on a carrier.

35. The process as claimed in Claim 31 wherein said metal acid phosphate catalyst is a Group IIA metal acid phosphate catalyst or a Group IVB metal acid phosphate catalyst.

36. The process as claimed in Claim 2 wherein said phosphorus-containing catalyst is a phosphorus acid or a phosphorus acid derivative compound.

37. The process as claimed in Claim 36 wherein the phosphorus acid or acid derivative compound is phosphoric acid, phophorous acid, boron phosphate, ferric phosphate, aluminum phosphate, hexaethylphosphorous triamide or hexamethylphosphorous triamide.

38. The method as claimed in Claim 2 wherein said phosphorus-containing catalyst is a phosphorus amide catalyst which has at least one phosphorus-nitrogen bond.

39. The method as claimed in Claim 38 wherein phosphorus amide catalyst has at least one -P-N-C-or -P-N-H bond.

40. The method as claimed in Claim 39 wherein the phosphorus amide catalyst is a phosphoramidous, phosphordiamidous, phosphoramidic, phosphordiamidic, phosphonamidous, or phophonamidic acid, ester, half-ester, anhydride, or metal salt, a triaminophosphine or triaminophosphine oxide, a phosphonic or phosphonous diamide, a phophinic amid, or a phosphonic amide.

41. The method as claimed in Claim 40 wherein the phosphorus amide catalyst is a phosphorous or phosphoric alkyleneamide.

42. The process as claimed in Claim 2 wherein, during said reaction, the pressure at which said reaction is conducted adjusted in order to control said mole ratio of said noncyclic polyalkylene polyamines to said cyclic polyalkylene polyamines, the increasing of said pressure resulting in an increase of said mole ratio and the lowering of said pressure resulting in a decrease of said mole ratio.

43. In a process for producing polyalkylene polyamines by contacting an alkyleneamine compound having at least two amino groups and an alkanolamine having at least one amino group, in a reaction zone, the improvement comprising increasing the pressure at which the reaction is conducted as needed throughout said reaction so as to maintain the mole ratio of said produced noncyclic

polyalkylene polyamines to said produced cyclic polyalkylene polyamines, said reaction being conducted in the presence of a catalytically effective amount of a phosphorous-containing catalyst at a temperature and pressure sufficient to form said polyalkylene polyamines.